# EUROPEAN PATENT APPLICATION

(11) **EP 1 974 743 A1**
(43) Date of publication of application: **01.10.2008**
(21) Application number: 07105074.4
(22) Date of filing: 28.03.2007
(51) Int. Cl.: A61K 39/05

(54) **Probiotics to Improve Gut Microbiota**

(71) Applicant: Nestec S.A., 1800 Vevey (CH)
(72) Inventor: Huber-Haag, Karl-Josef, 1009 Pully (CH); Fichot, Marie-Claire, 1807 Blonay (CH); Rochat, Florence, 1820 Montreux (CH); Sprenger, Nobert, 1073 Savigny (CH)
(74) Representative: Dixon, Sarah

(57) **Abstract**

The use of probiotic bacteria in the manufacture of a medicament or therapeutic nutritional composition for promoting the development of an early bifidogenic intestinal microbiota in infants delivered by caesarean section.

## Description

### Field of the Invention

This invention relates to the administration to infants delivered by Caesarean section of probiotic bacteria capable of promoting an early bifidogenic gut microflora.

### Background to the Invention

Immediately before birth, the gastro-intestinal tract of a baby is thought to be sterile. During the normal process of birth, it encounters bacteria from the digestive tract, skin and environment of the mother and starts to become colonised. The faecal microbiota of a healthy, vaginally-delivered, breast-fed infant of age 2 to 4 weeks which may be taken as the optimum microbiota for this age group is dominated by Bifidobacteria species with some Lactobacillus species and lesser amounts of Bacteroides such as *Bacteriodes fragilis* species, to the exclusion of potential pathogens such as Clostridia. After the completion of weaning at about 2 years of age, a pattern of gut microbiota that resembles the adult pattern becomes established.

It should be noted that, in the healthy, vaginally-delivered, breast-fed infant, Bifidobacteria form the basis of the microbiota accounting for 60-90 % of total bacteria in the infant gut. Breast feeding also promotes intestinal barrier development which, together with bifidobacterial domination leads to enhanced absorption and therefore utilisation of ingested nutrition.

Grönlund *et al* have studied the faecal microbiota of healthy infants born by caesarean section and compared it with that of a comparable group of infants born by vaginal delivery. They concluded that the gut flora of infants born by caesarean delivery may be disturbed for up to six months after the birth. Specifically they noted that the rates of colonisation by Bifidobacteria and Lactobacilli in the caesarean group reached the rates of colonisation in the vaginally delivered group only after one month and ten days respectively (Grönlund et al, "Fecal Microflora in Heathy Infants Born by Different Methods of Delivery: Permanent Changes in Intestinal Flora After Cesarean Delivery", Journal of Pediatric Gastroenterology and Nutrition, 28:19 - 25).

Other workers have suggested that this delayed/aberrant colonisation may have specific consequences in terms of the subsequent development of the infant and have linked these consequences to the differences in gut flora. For example, Negele *et al* found that caesarean delivery may be an additional risk factor for wheezing and allergic sensitisation to food allergens up to the age of two years (Negele et al "Mode of delivery and development of atopic disease during the first 2 years of life" Pediatr Allergy Immunol 2004:15:48 - 54). It has also been suggested that systemic low-grade inflammation and a sub-optimal gut microbiota may also be implicated in the development of obesity (Fantuzzi G. "Adipose tissue, adipokines, and inflammation" J Allergy Clin Immunol. 2005;115:911-919,. Bäckhed F, Ding H, Wang T, et al. "The gut microbiota as an environmental factor that regulates fat storage" Proc Natl Acad Sci USA. 2004;101:15718-15723).

Mother's milk is recommended for all infants. However, in some cases breast feeding is inadequate or unsuccessful for medical reasons or the mother chooses not to breast feed. Infant formulae have been developed for these situations.

In the recent past, certain strains of bacteria have attracted considerable attention because they have been found to exhibit valuable properties for man if ingested. In particular, specific strains of the genera Lactobacilli and Bifidobacteria have been found to be able to colonise the intestine, to reduce the capability of pathogenic bacteria to adhere to the intestinal epithelium, to have immunomodulatory effects and to assist in the maintenance of well-being. Such bacteria are sometimes called probiotics and it has already been proposed to add suitable probiotic bacteria to infant formulae.

Extensive studies have been carried out to identify new probiotic strains. For example, EP 0 199 535, EP 0 768 375, WO 97/00078, EP 0 577 903 and WO 00/53200 disclose specific strains of Lactobacilli and Bifidobacteria and their beneficial effects.

More recently, some concerns have been expressed about the addition of probiotic bacteria to infant formula which is intended as the sole source of nutrition for infants in the first six months of life. These concerns were summarized in the medical position paper from the ESPGHAN Committee on Nutrition entitled "Probiotic Bacteria in Dietetic Products for Infants" (Journal of Paediatric Gastroenterology and Nutrition, 38:365-374).

The intestinal microbiota plays an important role in the hydrolysis of indigestible oligosaccharides and polysaccharides to absorbable monosaccharides and activation of lipoprotein lipase by direct action on the villous epithelium. Further, it has recently been demonstrated that human milk contains not only oligosaccharides but also Bifidobacteria. At the same time, genomic studies have convincingly shown that Bifidobacteria present in the gut of breast-fed infants, such as *Bifidobacterium longum,* are specially equipped to utilize breast-milk oligosaccharides as nutrients. *Bifidobacterium longum* is also adapted to the conditions in the large intestine where energy harvest from slowly absorbable carbohydrates takes place.

In short, more and more evidence is emerging which suggests that the establishment of an appropriate intestinal microbiota early in life may be a significant in subsequent healthy development. At the same time the proportion of caesarean deliveries continues to increase reaching as much as 70% of all births in some countries. It is therefore clear that there is a need to provide a means to promote the rapid establishment of an appropriate intestinal microbiota in infants where this does not occur naturally. This need is particularly acute given the current practice of routinely administering prophylactic doses of antibiotics to pregnant women who undergo an elective caesarean delivery.

### Summary of the Invention

Accordingly the present invention provides the use of probiotic bacteria in the manufacture of a medicament or therapeutic nutritional composition for promoting the development of an early bifidogenic intestinal microbiota in infants delivered by caesarean section.

The invention further provides the use of probiotic bacteria in the manufacture of a medicament or therapeutic nutritional composition for reducing the risk of subsequent development of allergy in infants delivered by caesarean section.

In a further aspect, the invention provides the use of probiotic bacteria in the manufacture of a medicament or therapeutic nutritional composition for preventing or treating diarrhoea in infants delivered by caesarean section.

The invention extends to a method of promoting the development of an early bifidogenic intestinal microbiota in infants delivered by caesarean section comprising providing a therapeutic amount of probiotic bacteria to an infant born by caesarean section and in need of the same.

The invention further extends to a method of reducing the risk that an infant delivered by caesarean section will subsequently develop allergy comprising providing a therapeutic amount of probiotic bacteria to an infant born by caesarean section and in need of the same.

### Detailed Description of the Invention

In this specification, the following terms have the following meanings:-
"early bifidogenic intestinal microbiota" means for an infant up to the age of 12 months an intestinal microbiota which is dominated by Bifidobacteria such as *Bifidobacterium breve, Bifidobacterium infantis,* and *Bifidobacterium longum* to the exclusion of appreciable populations of such species as Clostridia and Streptococci and which is generally comparable with that found in a vaginally-delivered, breast fed infant of the same age.
"infant" means a child under the age of 12 months.
"probiotic" means microbial cell preparations or components of microbial cells with a beneficial effect on the health or well-being of the host. (Salminen S, Ouwehand A. Benno Y. et al "Probiotics: how should they be defined" Trends Food Sci. Technol. 1999:10 107-10).

All references to percentages are percentages by weight unless otherwise stated.

The probiotic bacteria may be any lactic acid bacteria or Bifidobacteria with established probiotic characteristics which are also capable of promoting the development of an early bifidogenic intestinal microbiota. Suitable probiotic lactic acid bacteria include *Lactobacillus rhamnosus* ATCC 53103 obtainable *inter alia* from Valio Oy of Finland under the trade mark LGG, *Lactobacillus rhamnosus* CGMCC 1.3724, *Lactobacillus reuteri* ATCC 55730 obtainable from Biogaia or *Lactobacillus paracasei* CNCM I-2116.

Suitable probiotic Bifidobacteria strains include *Bifidobacterium lactis* CNCM I-3446 sold *inter alia* by the Christian Hansen company of Denmark under the trade mark Bb12, *Bifidobacterium longum* ATCC BAA-999 sold by Morinaga Milk Industry Co. Ltd. of Japan under the trade mark BB536, the strain of *Bifidobacterium breve* sold by Danisco under the trade mark Bb-03, the strain of *Bifidobacterium breve* sold by Morinaga under the trade mark M-16V and the strain of *Bifidobacterium breve* sold by Institut Rosell (Lallemand) under the trade mark R0070. A mixture of suitable probiotic lactic acid bacteria and Bifidobacteria may be used.

A suitable daily dose of the probiotic bacteria is from 10e5 to 10e11 colony forming units (cfu), more preferably from 10e7 to 10e10 cfu.

The probiotic bacteria are preferably administered to the infant immediately after delivery and thereafter for at least the first two months of the life of the infant. More preferably, administration of the probiotic bacteria continues until the infant reaches six months of age.

The probiotic bacteria may be administered directly to the infant or, if the mother is breast-feeding, via the mother. If the probiotic bacteria are to be administered via the mother, they may be supplied to the mother as a supplement in the form of tablets, capsules, pastilles, chewing gum or a liquid for example. The supplement may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents, gel forming agents, antioxidants and antimicrobials. The supplement may also contain conventional pharmaceutical additives and adjuvants, excipients and diluents, including, but not limited to, water, gelatine of any origin, vegetable gums, ligninsulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like. In all cases, such further components will be selected having regard to their suitability for the intended recipient.

Alternatively, the probiotic bacteria may be administered to the mother in the form of a therapeutic nutritional composition. The composition may be a nutritionally complete formula.

A nutritionally complete formula for administration to pregnant women according to the invention may comprise a source of protein. Any suitable dietary protein may be used for example animal proteins (such as milk proteins, meat proteins and egg proteins); vegetable proteins (such as soy protein, wheat protein, rice protein, and pea protein); mixtures of free amino acids; or combinations thereof. Milk proteins such as casein and whey, and soy proteins are particularly preferred. The composition may also contain a source of carbohydrates and a source of fat.

If the formula includes a fat source in addition to the DHA, the fat source preferably provides 5% to 40% of the energy of the formula; for example 20% to 30% of the energy. A suitable fat profile may be obtained using a blend of canola oil, corn oil and high-oleic acid sunflower oil.

A source of carbohydrate may be added to the formula. It preferably provides 40% to 80% of the energy of the formula. Any suitable carbohydrate may be used, for example sucrose, lactose, glucose, fructose, corn syrup solids, maltodextrins, and mixtures thereof. Dietary fibre may also be added if desired. Dietary fibre passes through the small intestine undigested by enzymes and functions as a natural bulking agent and laxative. Dietary fibre may be soluble or insoluble and in general a blend of the two types is preferred. Suitable sources of dietary fibre include soy, pea, oat, pectin, guar gum, gum Arabic, fructooligosaccharides, galacto-oligosaccharides, sialyl-lactose and oligosaccharides derived from animal milks. A preferred fibre blend is a mixture of inulin with shorter chain fructo-oligosaccharides. Preferably, if fibre is present, the fibre content is between 2 and 40 g/l of the formula as consumed, more preferably between 4 and 10 g/l.

The formula may also contain minerals and micronutrients such as trace elements and vitamins in accordance with the recommendations of Government bodies such as the USRDA. For example, the formula may contain per daily dose one or more of the following micronutrients in the ranges given:- 300 to 500 mg calcium, 50 to 100 mg magnesium, 150 to 250 mg phosphorus, 5 to 20 mg iron, 1 to 7 mg zinc, 0.1 to 0.3 mg copper, 50 to 200 µg iodine, 5 to 15 µg selenium, 1000 to 3000 µg beta carotene, 10 to 80 mg Vitamin C, 1 to 2 mg Vitamin B1, 0.5 to 1.5 mg Vitamin B6, 0.5 to 2 mg Vitamin B2, 5 to 18 mg niacin, 0.5 to 2.0 µg Vitamin B12, 100 to 800 µg folic acid, 30 to 70 µg biotin, 1 to 5 µg Vitamin D, 3 to 10 IU Vitamin E.

One or more food grade emulsifiers may be incorporated into the formula if desired; for example diacetyl tartaric acid esters of mono- and di- glycerides, lecithin and mono- and di-glycerides. Similarly suitable salts and stabilisers may be included.

The formula is preferably enterally administrable; for example in the form of a powder for re-constitution with milk or water.
Alternatively, or in the case of infants who are not breast fed, the probiotic may be administered as a supplement, for example as a daily dose of 10e10 cfu dissolved in water and administered on a spoon.

For infants who are not breast fed, the probiotic bacteria may be conveniently administered in an infant formula.

An infant formula for use according to the present invention may contain a protein source in an amount of not more than 2.0 g/100kcal, preferably 1.8 to 2.0 g/100kcal. The type of protein is not believed to be critical to the present invention provided that the minimum requirements for essential amino acid content are met and satisfactory growth is ensured although it is preferred that over 50% by weight of the protein source is whey. Thus, protein sources based on whey, casein and mixtures thereof may be used as well as protein sources based on soy. As far as whey proteins are concerned, the protein source may be based on acid whey or sweet whey or mixtures thereof and may include alpha-lactalbumin and beta-lactoglobulin in whatever proportions are desired.

The proteins may be intact or hydrolysed or a mixture of intact and hydrolysed proteins. It may be desirable to supply partially hydrolysed proteins (degree of hydrolysis between 2 and 20%), for example for infants believed to be at risk of developing cows' milk allergy. If hydrolysed proteins are required, the hydrolysis process may be carried out as desired and as is known in the art. For example, a whey protein hydrolysate may be prepared by enzymatically hydrolysing the whey fraction in one or more steps. If the whey fraction used as the starting material is substantially lactose free, it is found that the protein suffers much less lysine blockage during the hydrolysis process. This enables the extent of lysine blockage to be reduced from about 15% by weight of total lysine to less than about 10% by weight of lysine; for example about 7% by weight of lysine which greatly improves the nutritional quality of the protein source.

The infant formula may contain a carbohydrate source. Any carbohydrate source conventionally found in infant formulae such as lactose, saccharose, maltodextrin, starch and mixtures thereof may be used although the preferred source of carbohydrates is lactose. Preferably the carbohydrate sources contribute between 35 and 65% of the total energy of the formula.

The infant formula may contain a source of lipids. The lipid source may be any lipid or fat which is suitable for use in infant formulas. Preferred fat sources include palm olein, high oleic sunflower oil and high oleic safflower oil. The essential fatty acids linoleic and α-linolenic acid may also be added as may small amounts of oils containing high quantities of preformed arachidonic acid and docosahexaenoic acid such as fish oils or microbial oils. In total, the fat content is preferably such as to contribute between 30 to 55% of the total energy of the formula. The fat source preferably has a ratio of n-6 to n-3 fatty acids of about 5:1 to about 15:1; for example about 8:1 to about 10:1.

The infant formula may also contain all vitamins and minerals understood to be essential in the daily diet and in nutritionally significant amounts. Minimum requirements have been established for certain vitamins and minerals. Examples of minerals, vitamins and other nutrients optionally present in the infant formula include vitamin A, vitamin B1, vitamin B2, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chloride, potassium, sodium, selenium, chromium, molybdenum, taurine, and L-carnitine. Minerals are usually added in salt form. The presence and amounts of specific minerals and other vitamins will vary depending on the intended infant population.

If necessary, the infant formula may contain emulsifiers and stabilisers such as soy lecithin, citric acid esters of mono- and di-glycerides, and the like.

The infant formula may optionally contain other substances which may have a beneficial effect such as fibres, lactoferrin, nucleotides, nucleosides, and the like.

Both the infant formula and the nutritional formula described above may be prepared in any suitable manner. For example, they may be prepared by blending together the protein, the carbohydrate source, and the fat source in appropriate proportions. If used, the emulsifiers may be included at this point. The vitamins and minerals may be added at this point but are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture. The temperature of the water is conveniently about 50°C to about 80°C to aid dispersal of the ingredients. Commercially available liquefiers may be used to form the liquid mixture. The liquid mixture is then homogenised; for example in two stages.

The liquid mixture may then be thermally treated to reduce bacterial loads, by rapidly heating the liquid mixture to a temperature in the range of about 80°C to about 150°C for about 5 seconds to about 5 minutes, for example. This may be carried out by steam injection, autoclave or by heat exchanger; for example a plate heat exchanger.

Then, the liquid mixture may be cooled to about 60°C to about 85°C; for example by flash cooling. The liquid mixture may then be again homogenised; for example in two stages at about 10 MPa to about 30 MPa in the first stage and about 2 MPa to about 10 MPa in the second stage. The homogenised mixture may then be further cooled to add any heat sensitive components; such as vitamins and minerals. The pH and solids content of the homogenised mixture are conveniently adjusted at this point.

The homogenised mixture is transferred to a suitable drying apparatus such as a spray drier or freeze drier and converted to powder. The powder should have a moisture content of less than about 5% by weight.

The selected probiotic bacteria may be cultured according to any suitable method and prepared for addition to the nutritional or infant formula by freeze-drying or spray-drying for example. Alternatively, bacterial preparations can be bought from specialist suppliers such as Christian Hansen and Valio already prepared in a suitable form for addition to food products such as nutritional and infant formulas. The probiotic bacteria may be added to the formula in an amount between 10e3 and 10e12 cfu/g powder, more preferably between 10e7 and 10e12 cfu/g powder.

The invention will now be further illustrated by reference to the following examples:-

### Example 1

An example of the composition of a suitable infant formula to be used in the present invention is given below

| Nutrient | per 100kcal | per litre |
|---|---|---|
| Energy (kcal) | 100 | 670 |
| Protein (g) | 1.83 | 12.3 |
| Fat (g) | 5.3 | 35.7 |
| Linoleic acid (g) | 0.79 | 5.3 |
| α-Linolenic acid (mg) | 101 | 675 |
| Lactose (g) | 11.2 | 74.7 |
| Minerals (g) | 0.37 | 2.5 |
| Na (mg) | 23 | 150 |
| K (mg) | 89 | 590 |
| Cl (mg) | 64 | 430 |
| Ca (mg) | 62 | 410 |
| P (mg) | 31 | 210 |
| Mg (mg) | 7 | 50 |
| Mn (µg) | 8 | 50 |
| Se (µg) | 2 | 13 |
| Vitamin A (µg RE) | 105 | 700 |
| Vitamin D (µg) | 1.5 | 10 |
| Vitamin E (mg TE) | 0.8 | 5.4 |
| Vitamin K1 (µg) | 8 | 54 |
| Vitamin C (mg) | 10 | 67 |
| Vitamin B1 (mg) | 0.07 | 0.47 |
| Vitamin B2 (mg) | 0.15 | 1.0 |
| Niacin (mg) | 1 | 6.7 |
| Vitamin B6 (mg) | 0.075 | 0.50 |
| Folic acid (µg) | 9 | 60 |
| Pantothenic acid (mg) | 0.45 | 3 |
| Vitamin B12 (µg) | 0.3 | 2 |
| Biotin (µg) | 2.2 | 15 |
| Choline (mg) | 10 | 67 |
| Fe (mg) | 1.2 | 8 |
| I (µg) | 15 | 100 |
| Cu (mg) | 0.06 | 0.4 |
| Zn (mg) | 0.75 | 5 |
| *L. rhamnosus* ATCC 53103 | 2.10⁷ cfu/g of powder, live bacteria | |

## Claims

1. The use of probiotic bacteria in the manufacture of a medicament or therapeutic nutritional composition for promoting the development of an early bifidogenic intestinal microbiota in infants delivered by caesarean section.

2. The use of probiotic bacteria in the manufacture of a medicament or therapeutic nutritional composition for reducing the risk of subsequent development of allergy in infants delivered by caesarean section.

3. The use of Claim 1 or 2, wherein the probiotic bacteria are lactic acid bacteria.

4. The use of Claim 3, wherein the lactic acid bacteria are of the strain *Lactobacillus rhamnosus* ATCC 53103, *Lactobacillus rhamnosus* CGMCC 1.3724, *Lactobacillus reuteri* ATCC 55730 or *Lactobacillus paracasei* CNCM I-2116.

5. The use of Claim 1 or 2, wherein the probiotic bacteria are Bifidobacteria.

6. The use of Claim 5, wherein the Bifidobacteria are of the strain *Bifidobacterium lactis* CNCM I-3446, *Bifidobacterium longum* ATCC BAA-999, *Bifidobacterium breve* Bb-03, *Bifidobacterium breve* M-16V or *Bifidobacterium breve* R0070.

7. The use of any preceding claim wherein the medicament or therapeutic nutritional composition is administered to the infant immediately after delivery and thereafter for at least 2 months.

8. The use of any preceding claim, wherein the medicament or therapeutic nutritional composition is administered to the infant for at least 6 months after delivery.

9. The use of any preceding claim, wherein the probiotic bacteria are administered to the infant via the breast-feeding mother.

10. The use of any of Claims 1 to 8, wherein the therapeutic nutritional composition is an infant formula.

11. The use of any preceding claim wherein the medicament comprises between 10e5 and 10e11 cfu of probiotic bacteria per daily dose.

12. The use of any of Claims 1 to 10 wherein the therapeutic nutritional composition comprises between 10e3 and 10e12 cfu of probiotic bacteria per gram of composition (dry weight).
